# FASCICULE DE BREVET EUROPEEN

(11) **EP 0 797 672 B1**
(45) Date de publication et mention de la délivrance du brevet: **11.01.2006**
(21) Numéro de dépôt: 96933496.0
(22) Date de dépôt: 07.10.1996
(51) Int. Cl.: C12N 15/74, C12N 15/31, C07K 14/315

(54) **UTILISATION D'UN SYSTEME DE SECRETION SEC-DEPENDANT POUR SECRETER DES PROTEINES NORMALEMENT SECRETEES PAR UN SYSTEME DE SECRETION SEC-INDEPENDANT**
VERWENDUNG EINES SEC-ABHÄNGIGEN SEKRETIONSSYSTEMS ZUR SEKRETION VON PROTEINEN, WELCHE GEWÖHNLICH DURCH EIN SEC-UNABHÄNGIGES SEKRETIONSSYSTEM SEKRETIERT WERDEN
USE OF A SEC-DEPENDENT SECRETION SYSTEM FOR SECRETING PROTEINS THAT ARE USUALLY SECRETED BY A SEC-INDEPENDENT SECRETION SYSTEM

(30) Priorité: 06.10.1995 FR 9511778
(43) Date de publication de la demande: 01.10.1997
(73) Titulaire: SKW BIOSYSTEMS, 92100 Boulogne-Billancourt (FR)
(72) Inventeur: FAYARD, Blandine, NL-9688 RC Drieborg (NL); KOK, Jan, NL-9714 HL Groningen (NL); VENEMA, Gerhardus, NL-9751 NN Haren (NL); BIGRET, Marc, F-94170 Le Perreux (FR); PREVOTS, Fabien, F-31400 Toulouse (FR)
(74) Mandataire: Gillard, Marie-Louise
(86) Numéro de dépôt international: PCT/FR1996/001560
(87) Numéro de publication internationale: WO 1997/013863

(56) Documents cités:
- EP-A- 0 244 042
- EP-A- 0 455 280
- WO-A-91/19802
- WO-A-92/04451
- FEMS MICROBIOLGY REVIEWS, vol. 88, 1992, pages 73-92, XP002007283 M. VAN DE GUCHTE ET AL: "Gene expression in Lactococcus lactis"
- APPLIED AND ENVIRONMENTAL MICROBIOLOGY, vol. 54, no. 1, Janvier 1988, pages 231-238, XP000574091 J. KOK ET AL: "Nucleotide sequence of the cell wall proteinase gene of Streptococcus cremoris Wg2" cité dans la demande
- APPLIED AND ENVIRONMENTAL MICROBIOLOGY, vol. 58, no. 2, Février 1992, pages 572-577, XP000574082 M. VAN BELKUM: "Cloning, sequencing, and expression in Escherichia coli og lcnB, a third bacteriocin determinant for lactococcal....."
- G. JUNG ET H-G SAHL: "Nisin and novel lantibiotics" 1991 , ESCOM , LEIDEN XP002007284 voir page 250 - page 258

## Description

La présente invention a pour objet l'utilisation d'un système de sécrétion Sec-dépendant pour sécréter des protéines normalement sécrétées par un système de sécrétion Sec-indépendant. Elle a également pour objet les bactéries lactiques contenant ce système de sécrétion Sec-dépendant, en particulier les lactocoques appartenant à l'espèce *Lactococcus lactis*, l'utilisation de certaines souches de ces lactocoques pour transférer ledit système de sécrétion à des souches d'intérêt industriel, en particulier dans l'industrie laitière, et l'emploi de certaines souches *Lactococcus lactis* pour obtenir ce système de sécrétion.

La sécrétion de protéines chez les bactéries suit en général deux voies. La plus connue est le transport Sec-dépendant. Selon cette voie, la protéine est exportée grâce à la présence en position N-terminale d'un peptide-signal qui est clivé dans la protéine sécrétée. Chez *E.coli*, plusieurs protéines impliquées dans cette sécrétion ont été mises en évidence, en particulier SecA, SecY et SecE. Chez *L.lactis,* le gène *sec*Y a été mis en évidence : il possède une homologie importante avec le gène *sec*Y de *E.coli* indiquant que ce système de sécrétion est très vraisemblablement présent également chez *L.lactis* (Koivula et al., 1991, FEBS Lett. 228 : 114-118).

La seconde voie de sécrétion chez les bactéries, ne dépendant pas des protéines Sec, implique des translocateurs transmembranaires, appelés famille des protéines A, B, C (Pugsley, 1993, Microbiol. Rev. 57 : 50-108).

Chez *L.lactis* des translocateurs ont été mis en évidence ; ils sont impliqués dans la sécrétion des bactériocines LcnA, LcnB, LcnM1 et des antibiotiques lacticin 481, nisine A et nisine Z (de Vos et Simons, Genetics and Biotechnology of L.A.B., Blackie Academic and Professional, 1994).

L'étude de ces deux voies de sécrétion chez *L.lactis* a été mise à profit pour mettre au point différents systèmes de sécrétion de protéines hétérologues. L'ensemble des vecteurs de sécrétion mis au point est contenu dans la revue de de Vos et Simons, Genetics and Biotechnology of L.A.B., Blackie Academic and Professional, 1994.

Les bactériocines mises en évidence chez les bactéries lactiques sont presque toutes sécrétées par le système Sec-indépendant (Dodd et Gasson, Genetics and Biotechnology of L.A.B., Blackie Academic and Professional, 1994). Cependant, pour des raisons d'augmentation de la sécrétion de ces bactériocines, il peut être important de faire sécréter ces bactériocines par un autre système de sécrétion.

Des auteurs ont récemment montré que la Divergicin **A**, bactériocine produite par *Carnobacterium divergens*, est sécrétée via un système Sec-dépendant (Worobo et al., J. Bacteriol. 177 : 3143-3149.). Il n'a toutefois jamais été montré chez les bactéries lactiques qu'une bactériocine sécrétée par un système Sec-indépendant pouvait être sécrétée par un système Sec-dépendant.

La Demanderesse a effectué des travaux dans ce domaine et a trouvé de manière surprenante que l'on peut utiliser un système de sécrétion Sec-dépendant, pour sécréter des protéines normalement sécrétées par un système de sécrétion Sec-indépendant.

Selon l'invention, le système de sécrétion Sec-dépendant est utilisé en combinaison avec la séquence d'ADN codant pour une protéine mature et normalement sécrétée par un système Sec-indépendant, le promoteur P32 de lactocoque, la séquence-signal sp de la protéase de lactocoque et le terminateur du gène de l'immunité à LcnB (lciB) pour sécréter ladite protéine mature normalement sécrétée par un système de sécrétion Sec-indépendant.

Ainsi l'invention a également pour objet la construction d'ADN pour la sécrétion par un système Sec-dépendant de protéines normalement sécrétées par un système Sec-indépendant, qui comprend le promoteur P32 de lactocoque, la séquence-signal sp de la protéase de lactocoque, la séquence d'ADN codant pour une protéine mature et normalement sécrétée par un système Sec-indépendant, le terminateur du gène de l'immunité à LcnB (lciB). Cette construction d'ADN peut être présente sur un vecteur d'expression, tel qu'un plasmide, dans l'ADN génomique ou tout autre fragment d'ADN.

Selon l'invention, on peut utiliser un système de sécrétion Sec-dépendant quelconque en combinaison avec un gène codant pour une protéine d'intérêt qui est normalement sécrétée par un système Sec-indépendant.

A titre d'exemples de gènes codant pour une protéine d'intérêt, on peut utiliser notamment les gènes codant pour les bactériocines en particulier la bactériocine *Lcn*B, qui est une bactériocine sécrétée par *L.lactis* sous la dépendance d'un système de sécrétion Sec-indépendant (Van Belkum et al., 1992, Applied Environ. Microbiol., 58 : 572-577).

L'invention a également pour objet les bactéries lactiques de préférence *L.lactis* sécrétant une ou plusieurs bactériocines qui contiennent une construction, selon l'invention, telle que définie ci-dessus, qui permet la sécrétion de ces bactériocines. Cette construction peut être introduite dans les bactéries lactiques par conjugaison, transformation, fusion de protoplastes ou par une autre méthode de transfert de gènes.

Les bactéries lactiques qui peuvent avantageusement être transformées à l'aide de la construction selon l'invention sont par exemple *Lactococcus lactis ssp cremoris, Lactococcus lactis ssp lactis, Lactococcus lactis ssp lactis var diacetylactis*.

Ces souches ainsi transformées peuvent être utilisées pour transmettre par conjugaison, transformation, transduction, fusion de protoplastes ou une autre méthode de transfert de gènes, une construction selon l'invention à une souche d'intérêt industriel. Cette construction peut être portée par un plasmide ou par une autre partie du génome de la bactérie.

L'invention a également trait aux souches d'intérêt industriel sécrétant des protéines ainsi obtenues telles que par exemple les bactériocines. Ces bactéries sécrétant des bactériocines peuvent être utilisées afin d'inhiber le développement de bactéries pathogènes, de lyser des bactéries afin que leur contenu enzymatique participe à l'affinage des produits fermentés par ces bactéries, ou afin d'avoir des bactéries pour tout usage impliquant une sécrétion de bactériocines par une construction selon l'invention.

L'invention sera mieux comprise à l'aide des exemples illustratifs mais non limitatifs ci-après.

Une grande partie de l'ensemble des techniques décrites dans ces exemples, bien connues de l'homme de l'art, est exposée en détail dans l'ouvrage de Sambrook, Fritsch et Maniatis : "Molecular cloning ; a laboratory manual" publié en 1989 par les éditions Cold Spring Harbor Press à New York (2e édition).

La description ci-après sera mieux comprise à l'aide des figures 1 à 3 ci-après qui représentent respectivement :
Figure 1 : Construction du vecteur pSV123
Figure 2 : Construction du vecteur de sécrétion pSV
Figure 3 : Construction du plasmide pSV*Lcn*B permettant la sécrétion de *Lcn*B

### Exemple 1 : Construction du plasmide vecteur de sécrétion pSV

Afin de pouvoir sécréter une bactériocine dont la sécrétion est Sec-indépendante, il a fallu au préalable construire un vecteur de sécrétion Sec-dépendant. Cette construction a été faite de la façon suivante :

Pour chacun des clonages où cela a été nécessaire, la technique PCR a été utilisée dans les conditions suivantes : 1 minute à 94°C (dénaturation), 2 minutes à 45°C (hybridation) et 2 minutes à 73°C (polymérisation), 30 cycles. Les oligonucléotides ont été synthétisés avec un synthétiseur Applied Biosystems 381A DNA (Applied Biosystems).

Pour la construction du vecteur de sécrétion pSV, les oligonucléotides A, B, C, D ont été utilisés.

Les séquences de ces oligonucléotides sont les suivantes (les sites de restriction utilisés pour le clonage sont soulignés, le RBS dans l'oligonucléotide B apparaît en gras) :

Des produits de PCR (Polymerase Chain Reaction) contenant le promoteur et le site de liaison aux ribosomes RBS (Ribosome Binding Site) de l'ORF32 (Open Reading Frame = phase de lecture ouverte) ont été obtenus à partir du plasmide pMG36c (Van de Guchte et al., 1989, Applied Environ. Microbiol., 55 : 224-228) qui dérive du plasmide pMG36e utilisé ci-après.

La séquence-signal sp de la protéase de lactocoque PrtP a été obtenue à partir du gène *prtP* présent dans le plasmide pGKV500 (Kok et al, 1988, Applied Environ. Microbiol, 54 : 221-228).

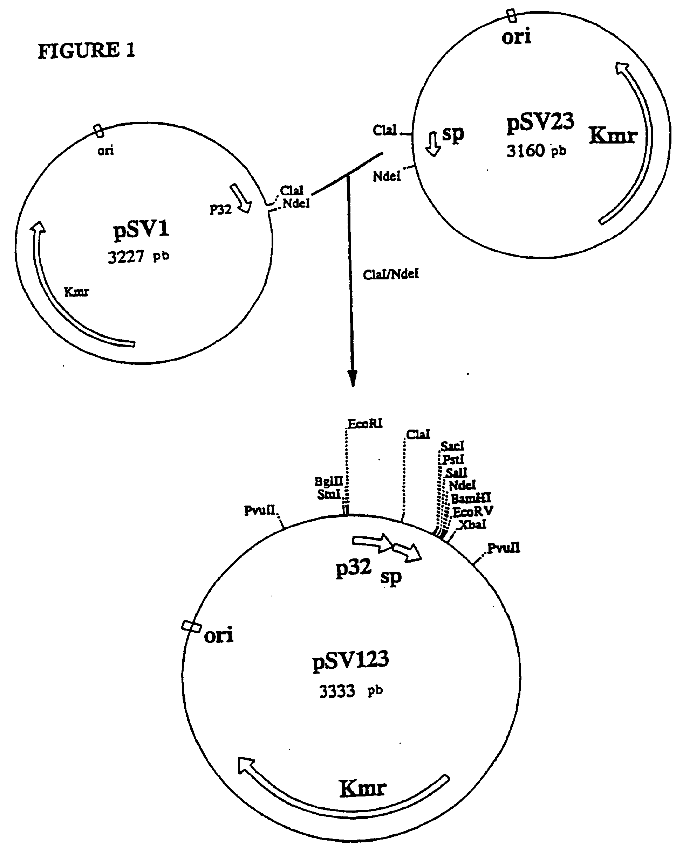

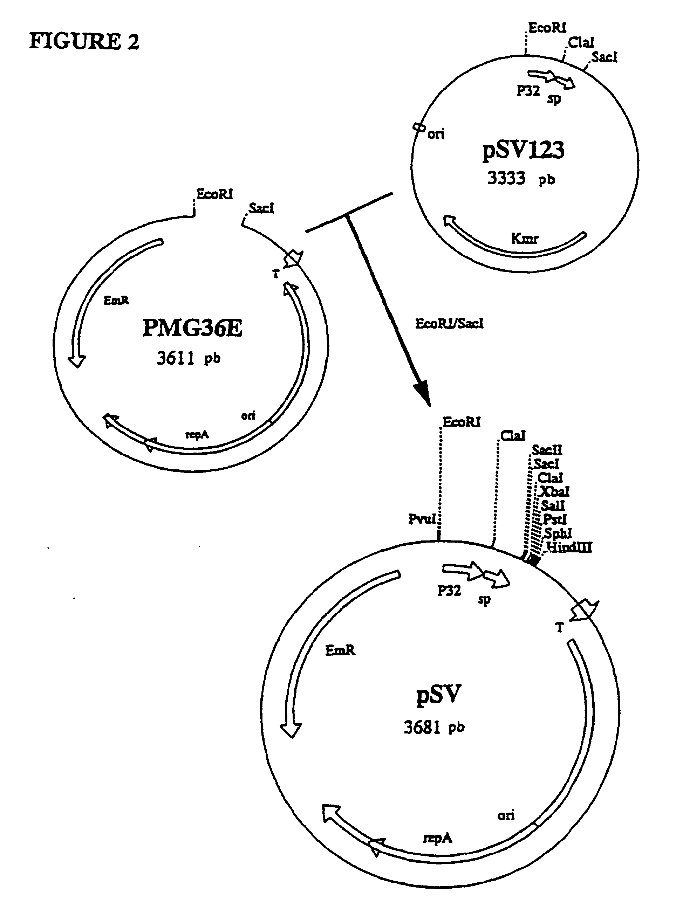

Ces produits de PCR ont été clonés après digestion par les enzymes de restriction BgIII et PstI, dans le plasmide pUK21 (Vieira et Messing, 1991, Gene, 100 : 189-194), pour donner les plasmides pSV1 et pSV23 (figure 1). Ensuite, le plasmide pSV23 a été digéré par les enzymes de restriction à ClaI et NdeI, et le fragment contenant la séquence-signal a été cloné dans le plasmide pSV1 préalablement digéré par les enzymes de restriction ClaI et Ndel, pour donner le plasmide pSV123 (figure 1). Enfin, le fragment *Eco*RI-*Sac*I de pSV123, contenant le promoteur P32 et la séquence-signal sp, a été cloné dans le plasmide pMG36e (Van de Guchte et al., 1989, Applied Environ. Microbiol., 55 : 224-228), pour donner le vecteur de sécrétion pSV (figure 2).

### Exemple 2 : Construction du plasmide pSVLcnB impliqué dans la sécrétion de la bactériocine LcnB

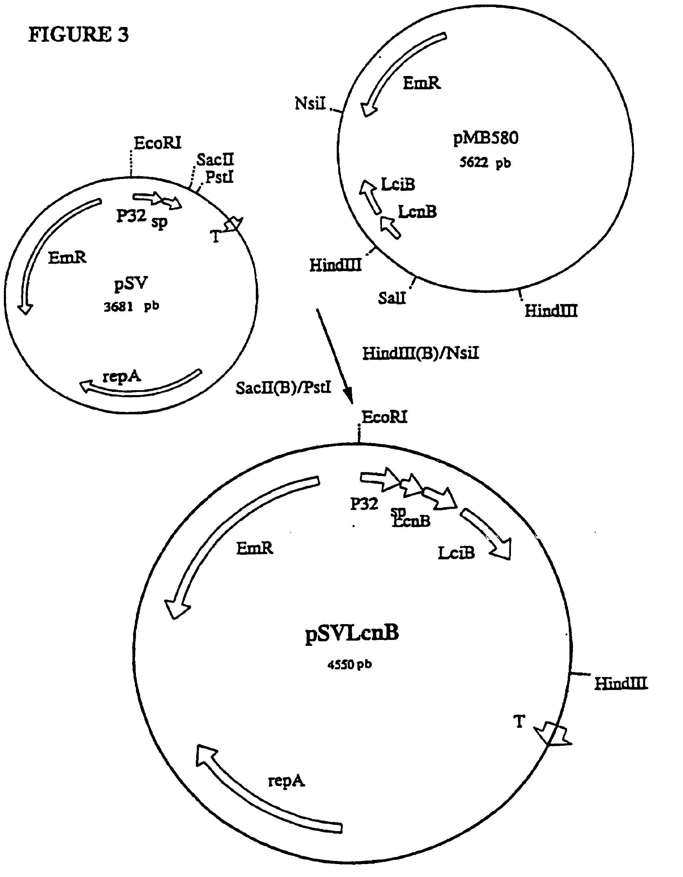

Le plasmide pMB580 (plasmide conférant la résistance à l'érythromycine, dérivé du plasmide pGK210, contenant l'opéron de la lactococcine B, c'est-à-dire *Lcn*B et *lci*B - Van Belkum et al, 1992 Applied Environ. Microbiol., 58 : 572-577) a été digéré par les enzymes de restriction à *Nsi*I et *Hind*III. L'extrémité *Hind*III a été hydrolysée pour la transformer en extrémité franche. Le fragment *Hind*III-*Nsi*I a été ligaturé au plasmide pSV préalablement digéré par les enzymes de restriction à PstI et SacII, et dont l'extrémité SacII a été hydrolysée pour la transformer en extrémité franche, pour donner le plasmide pSV*Lcn*B (figure 3). La séquence résultante en acides aminés de ce dérivé de *Lcn*B avec la séquence-signal de la protéine PrtP est la séquence ci-après :

### Exemple 3 : Sécrétion de LcnB par un système de sécrétion Sec-dépendant

Le plasmide dérivé du plasmide pSV (pSV*Lcn*B), contenant le gène de structure de la lactococcine LcnB (*lcn*B) et le gène de l'immunité à *Lcn*B (*lci*B), a servi à transformer la souche *E.coli* JM101 (Maniatis et al., 1982, Molecular Cloning : a laboratory manual. Cold Spring Harbor, New York, Cold Spring Harbor Laboratory), la souche *Lactococcus lactis* MG1363 (Gasson, 1983, J. Bacteriol., 154 : 1-9) et la souche *Lactococcus lactis* IL1403 (Chopin et al., 1984, Plasmid, 11, : 260-263). Dans ces constructions, l'extension N-terminale de la prébactériocine a été remplacée par le peptide signal de PrtP (figures 1, 2 et 3).

Afin de tester l'activité bactériocine, les souches ont été recouvertes par une surcouche de la souche IL1043 contenant, soit pMG36e, soit pMG36c. Ce test a été réalisé de la façon suivante :

Des colonies contenant la construction (pSV*Lcn*B) ont été incubées 16 h à 30° C sur des boîtes de M17 (Terzaghi et Sandine, 1975, Appl. Microbiol, 29: 807-813) + glucose 5 g/l + agar 5 g/l. Après exposition à des vapeurs de chloroforme pendant 20 minutes, les boîtes ont été partiellement séchées à l'air pour éliminer les traces résiduelles de chloroforme. Puis, on a étalé sur ces boîtes 4 ml de gélose M17 + glucose 5 g/l + agar 7 g/l inoculé avec 40 µl de culture de nuit de la souche indicatrice *L.lactis* IL1403, contenant pMG36e ou pMG36c. Après incubation à 30° C pendant 12 à 18 h, les boîtes ont été analysées pour les zones d'inhibition (halos) autour des clones. Les essais de surcouche sur des gels de tricine ont été effectués selon le mode opératoire décrit par Van Belkum et al., 1992, dans Applied Environ. Microbiol., 58 : 572-577.

Les résultats ont montré que l'on a un halo d'inhibition autour des clones contenant pSV*Lcn*B. Ce halo est plus large autour des clones contenant pSV*Lcn*Bc. Cela indique que la bactériocine LcnB a bien été sécrétée grâce à pSV*Lcn*B et pSV*Lcn*Bc. Afin de confirmer ce résultat, et surtout de savoir si l'activité bactériocine est due à une forme maturée (sans le peptide signal sp) ou non maturée (avec le peptide signal sp), des sumageants de culture de souche IL1403 contenant pSV*Lcn*B ont été soumis à une précipitation au sulfate d'ammonium, et le précipité a été testé sur gel d'électrophorèse tricine SDS-PAA. Les gels ont ensuite été examinés pour l'activité bactéricide. Les résultats montrent que l'on a une inhibition de croissance dans la zone des 3, 4 KDa, correspondant à la taille de la bactériocine LcnB maturée, c'est-à-dire débarrassée de la séquence-signal sp. Comme la souche *Lactococcus lactis* MG1363 ne possède pas le système de sécrétion Sec-indépendant des bactériocines, la bactériocine LcnB produite grâce au plasmide pSV*Lcn*B a bien été sécrétée via le système de sécrétion Sec-dépendant, en utilisant la séquence-signal sp de la protéase de lactocoque PrtP.

### LISTE DE SEQUENCES

(1) INFORMATIONS GENERALES:
   (i) DEPOSANT:
      (A) NOM: BIOGHURT BIOGARDE GmbH & Co. KG
      (B) RUE: Lise-Meitner-Strasse 34
      (C) VILLE: FREISING
      (E) PAYS: ALLEMAGNE
      (F) CODE POSTAL: 85354
      (G) TELEPHONE:
      (H) TELECOPIE:
   (ii) TITRE DE L' INVENTION: Utilisation d'un système de sécrétion Sec-dépendant pour sécréter des protéines normalement sécrétées par un système de sécrétion Sec-indépendant, bactéries les contenant et leur utilisation
   (iii) NOMBRE DE SEQUENCES: 5
   (iv) FORME DECHIFFRABLE PAR ORDINATEUR:
      (A) TYPE DE SUPPORT: Floppy disk
      (B) ORDINATEUR: IBM PC compatible
      (C) SYSTEME D'EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 33 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: OUI
   (xi) DESCRIPTION DE LA SEQUENCE: SEQ ID NO: 1:
   AGATCTAGAT CTGAATTCGG TCCTCGGGAT ATG 33
(2) INFORMATIONS POUR LA SEQ ID NO: 2:
   (i) CARACTERISTIQUES DE LA SEQUENCE:
      (A) LONGUEUR: 44 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: OUI
   (xi) DESCRIPTION DE LA SEQUENCE: SEQ ID NO: 2:
   CTGCAGCTGC AGCATCGATA AATTCCTCCG AATATTTTTT TACC 44
(2) INFORMATIONS POUR LA SEQ ID NO: 3:
   (i) CARACTERISTIQUES DE LA SEQUENCE:
      (A) LONGUEUR: 49 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: OUI
   (xi) DESCRIPTION DE LA SEQUENCE: SEQ ID NO: 3:
   AGATCTAGAT CTATCGATGC AAAGGAAAAA GAAAGGGCTT TCGATCTTG 49
(2) INFORMATIONS POUR LA SEQ ID NO: 4:
   (i) CARACTERISTIQUES DE LA SEQUENCE:
      (A) LONGUEUR: 44 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: OUI
   (xi) DESCRIPTION DE LA SEQUENCE: SEQ ID NO: 4:
   CTGCAGCTGC AGAGCTCGCC GCGGCCTTTG CTTGGATTTC GCCG 44
(2) INFORMATIONS POUR LA SEQ ID NO: 5:
   (i) CARACTERISTIQUES DE LA SEQUENCE:
      (A) LONGUEUR: 80 acides aminés
      (B) TYPE: acide aminé
      (C) NOMBRE DE BRINS:
      (D) CONFIGURATION: linéaire
   (ii) TYPE DE MOLECULE: peptide
   (xi) DESCRIPTION DE LA SEQUENCE: SEQ ID NO: 5:
   Met Gln Arg Lys Lys Lys Gly Leu Ser Ile Leu Leu Ala Gly Thr Val
   1               5                   10                  15
   Ala Leu Gly Ala Leu Ala Val Leu Pro Val Gly Glu Ile Gln Ala Lys
               20                  25                  30
   Ala Ser Leu Gln Tyr Val Met Ser Ala Gly Pro Tyr Thr Trp Tyr Lys
           35                  40                  45
   Asp Glu Arg Thr Gly Lys Thr Glu Cys Lys Gln Thr Ile Asp Thr Ala
       50                  55                  60
   Ser Tyr Thr Phe Gly Val Met Ala Glu Gly Trp Gly Lys Thr Phe His
   65                  70                  75                  80

## Revendications

1. Utilisation d'un système de sécrétion Sec-dépendant en combinaison avec la séquence d'ADN codant pour une protéine mature et normalement sécrétée par un système Sec-indépendant, le promoteur P32 de lactocoque, la séquence-signal sp de la protéase de lactocoque et le terminateur du gène de l'immunité à LcnB (lciB) pour sécréter ladite protéine mature normalement sécrétée par un système de sécrétion Sec-indépendant.

2. Utilisation selon la revendication 1, **caractérisée en ce que** la séquence d'ADN codant pour la protéine mature est la séquence d'ADN codant pour une bactériocine.

3. Utilisation selon l'une des revendications 1 ou 2, **caractérisée en ce que** la bactériocine est la bactériocine LcnB.

4. Construction d'ADN pour la sécrétion par un système Sec-dépendant d'une protéine normalement sécrétée par un système Sec-indépendant, **caractérisée en ce qu'**elle comprend le promoteur P32 de lactocoque, la séquence-signal sp de la protéase de lactocoque, la séquence d'ADN codant pour une protéine mature et normalement sécrétée par un système Sec-indépendant, le terminateur du gène de l'immunité à LcnB (lciB).

5. Construction selon la revendication 4, **caractérisée en ce que** la séquence d'ADN codant pour la protéine est la séquence d'ADN codant pour une bactériocine.

6. Construction selon la revendication 5, **caractérisée en ce que** la bactériocine est la bactériocine LcnB.

## Patentansprüche

1. Verwendung eines Sec-abhängigen Sekretionssystems in Kombination mit der DNA-Sequenz, die ein reifes und normalerweise von einem Sec-unabhängigen System sezerniertes Protein codiert, dem Lactocuccus-Promotor P32, der Signalsequenz sp der Lactococcus-Protease und dem Terminator des Gens der Immunität gegen LcnB (lciB) zur Sekretion des reifen Proteins, das normalerweise von einem Sec-unabhängigen Sekretionssystem sezerniert wird.

2. Verwendung nach Anspruch 1, **dadurch gekennzeichnet, dass** die DNA-Sequenz, die das reife Protein codiert, die DNA-Sequenz ist, die ein Bacteriocin codiert.

3. Verwendung nach Anspruch 1 oder 2, **dadurch gekennzeichnet, dass** das Bacteriocin das Bacteriocin LcnB ist.

4. DNA-Konstrukt zur Sekretion eines Proteins, das normalerweise durch ein Sec-abhängiges System sezerniert wird, durch ein Sec-abhängiges System, **dadurch gekennzeichnet, dass** es den Lactococcus-Promotor P32, die Signalsequenz sp der Lactococcus-Protease, die DNA-Sequenz, die ein reifes und normalerweise von einem Sec-unabhängigen System sezerniertes Protein codiert, und den Terminator des Gens der Immunität gegen LcnB (lciB) enthält.

5. Konstrukt nach Anspruch 4, **dadurch gekennzeichnet, dass** die DNA-Sequenz, die das Protein codiert, die DNA-Sequenz ist, die ein Bacteriocin codiert.

6. Konstrukt nach Anspruch 5, **dadurch gekennzeichnet, dass** das Bacteriocin das Bacteriocin LcnB ist.

## Claims

1. Use of a Sec-dependent secretion system in combination with the DNA sequence coding for a mature protein normally secreted by a Sec-independent system, the P32 promoter of lactococcus, the sp signal sequence of the protease of lactococcus and the terminator of the LcnB immunity gene (lciB) for secreting the mature protein normally secreted by a Sec-independent secretion system.

2. Use according to claim 1, **characterized in that** the DNA sequence coding for the mature protein is the DNA sequence coding for a bacteriocin.

3. Use according to claim 1 or 2, **characterized in that** the bacteriocin is the bacteriocin LcnB.

4. DNA construction for the secretion, by a Sec-dependent system, of a protein normally secreted by a Sec-independent system, **characterized in that** it comprises the P32 promoter of lactococcus, the sp signal sequence of the protease of lactococcus, the DNA sequence coding for a mature protein normally secreted by a Sec-independent system, and the terminator of the LcnB immunity gene (lciB).

5. Construction according to claim 4, **characterized in that** the DNA sequence coding for the protein is the DNA sequence coding for a bacteriocin.

6. Construction according to claim 5, **characterized in that** the bacteriocin is the bacteriocin LcnB.
